# EUROPEAN PATENT SPECIFICATION

(11) **EP 3 169 789 B1**
(45) Date of publication and mention of the grant of the patent: **01.09.2021**
(21) Application number: 15738628.5
(22) Date of filing: 16.07.2015
(51) Int. Cl.: C12P 5/02, C12P 7/52, C12P 7/40, C25B 3/29

(54) **PROCESS FOR PRODUCING ALKANES USING MICROORGANISMS COMBINED WITH KOLBE SYNTHESIS**
VERFAHREN ZUR HERSTELLUNG VON ALKANEN MITTELS MIKROORGANISMEN IN KOMBINATION MIT KOLBE-SYNTHESE
PROCÉDÉ POUR LA PRODUCTION D'ALCANES À L'AIDE DE MICRO-ORGANISMES COMBINÉS AVEC UNE SYNTHÈSE DE KOLBE

(30) Priority: 17.07.2014 EP 14177491
(43) Date of publication of application: 24.05.2017
(73) Proprietor: Evonik Operations GmbH, 45128 Essen (DE)
(72) Inventor: HAAS, Thomas, 48161 Münster (DE); PAULMANN, Uwe, 59348 Lüdinghausen (DE); BECK, Simon, 48163 Münster (DE)
(74) Representative: Evonik Patent Association
(86) International application number: PCT/EP2015/066275
(87) International publication number: WO 2016/008979

(56) References cited:
- WO-A1-2012/099603
- WO-A2-2007/095215
- WO-A2-2011/066634
- US-A- 3 992 268
- US-A1- 2011 111 475
- LEVY P F ET AL: "Kolbe Electrolysis of Mixtures of Aliphatic Organic Acids", JOURNAL OF THE ELECTROCHEMICAL SOCIETY,, vol. 131, no. 4, 1 April 1984 (1984-04-01) , pages 773-777, XP001328869,
- LEVY P F ET AL: "Biorefining of biomass to liquid fuels and organic chemicals", ENZYME AND MICROBIAL TECHNOLOGY, STONEHAM, MA, US, vol. 3, no. 3, 1 July 1981 (1981-07-01), pages 207-215, XP023679329, ISSN: 0141-0229, DOI: 10.1016/0141-0229(81)90087-9 [retrieved on 1981-07-01]
- H. Seedorf ET AL: "The genome of Clostridium kluyveri, a strict anaerobe with unique metabolic features", Proceedings of the National Academy of Sciences, vol. 105, no. 6, 12 February 2008 (2008-02-12), pages 2128-2133, XP055499198, US ISSN: 0027-8424, DOI: 10.1073/pnas.0711093105

## Description

### FIELD OF THE INVENTION

The present invention related to a method of synthesising branched, unbranched and long chained alkanes comprising the production of at least one carboxylic acid using at least one microorganism from a carbon source comprising ethanol in combination with other carbon sources, and performing Kolbe electrolysis on the carboxylic acid to produce the alkanes. In particular, the method is a biotechnological method.

### BACKGROUND OF THE INVENTION

Alkanes are saturated hydrocarbons that have various applications depending on the number of carbon atoms and on the structure of the alkane (i.e. branched, linear, cyclic etc.). The first four alkanes (CH₄ to C₄H₈) are used mainly for heating and cooking purposes and in some countries for electricity generation. Pentane, hexane, heptane and octane are reasonably volatile liquids. They are usually used as fuels in internal combustion engines, as they vaporise easily on entry into the combustion chamber without forming droplets, which would impair the uniformity of the combustion. For this function, branched-chain alkanes are preferred as they are much less prone to premature ignition, which causes knocking, compared to their straight-chain counterparts. This propensity to premature ignition is measured by the octane rating of the fuel, where for example, 2,2,4-trimethylpentane (isooctane) has an arbitrary value of 100, and heptane has a value of zero. Apart from their use as fuels, this group of alkanes are also good solvents for nonpolar substances.

Alkanes from nonane onwards, for instance, to hexadecane (an alkane with sixteen carbon atoms) are liquids of higher viscosity, less and less suitable for use in gasoline. They form instead the major part of diesel and aviation fuel. Diesel fuels are characterized by their cetane number, cetane being an old name for hexadecane.

Alkanes from hexadecane upwards form the most important components of fuel oil and lubricating oil. In the latter function, they work at the same time as anti-corrosive agents, as their hydrophobic nature means that water cannot reach the metal surface. Many solid alkanes find use as paraffin wax, for example, in candles. This should not be confused however with true wax, which consists primarily of esters. Alkanes with a chain length of approximately 35 or more carbon atoms are found in bitumen, used, for example, in road surfacing. However, the higher alkanes have little value and are usually split into lower alkanes by cracking.

Accordingly, alkanes of all lengths are very useful in our day to day world. Currently, traditional manufacture of alkanes uses petroleum based intermediates. However, these alkanes are obtained by cracking gasoline or petroleum which is bad for the environment. Also, since the costs for these alkanes will be linked to the price of petroleum, with the expected increase in petroleum prices in the future, alkane prices may also increase relative to the increase in the petroleum prices.

Accordingly, it is desirable to find other methods of producing alkanes from more sustainable raw materials, other than purely petroleum based raw materials which also cause less damage to the environment.

There are numerous efforts underway to generate renewable fuels including alkanes from sustainable raw materials, such as synthesis gas. One method known in the art is to generate biodiesel fuel (predominantly fatty acid ethyl or methyl esters) from triglycerides. Another approach is to use the glycerol to form glycerol ethers, which can be added to biodiesel and/or diesel fuel. However, each of these methods has its' own disadvantages. There is thus still no known method of efficiently producing middle or long chained alkanes from renewable fuels including synthesis gas.

Kuhry et al. (US 2011/0111475 A1, WO 2012/099603 A1) disclose a method for producing hydrocarbon and hydrogen fuels by fermenting biomass material in a mixed culture of microorganisms derived from rumen contents and subjecting the resulting volatile fatty acids to a Kolbe electrolysis (Levy et al., Journal of the Electrochemical Society, Vol. 131, 1984, pp. 773-777). Bradin (WO 2007/095215 A2) also provides a method for the production of gasoline from fermentable feedstocks, wherein essentially in a first step butanoic acid is produced by fermenting sugars using bacteria or yeast and in a subsequent step the butanoic acid obtained is subjected to Kolbe or photo-Kolbe electrolysis. Pereira et al. (WO 2011/066634 A2) propose a method for the production of olefins by producing carboxylic acids by fermentation of sugars and subjecting the so produced carboxylic acids an anodic electro-decarboxylation. Antos (US 3 992 268) discloses a process for the production of hydrocarbonaceous materials by reacting waste materials with a microorganism to produce organic products and treating the resulting organic products by electrolysis to form hydrocarbonaceous materials and carbon dioxide (see also Levy et al., Enzyme Microb. Technol., 1981, Vol. 3, pp. 207-215).

### DESCRIPTION OF THE INVENTION

The present invention provides a process of producing middle or long chained alkanes from renewable fuels. In particular, the method of the present invention uses ethanol as carbon source in combination with at least one other carbon source selected from the group consisting of acetate, propionate, butyrate, isobutyrate, valerate and hexanoate to produce branched, linear and/or cyclic alkanes using at least a two-step biotechnological process. The electrolysis is performed by Kolbe electrolysis and carbon dioxide may be produced as a by-product.

The present invention provides a method of producing at least one alkane the method comprising,
- producing at least one carboxylic acid from a carbon source using a microorganism, and
- performing Kolbe electrolysis on the carboxylic acid to produce the alkane,
wherein the alkane comprises at least 6 carbon atoms and the carboxylic acid comprises at least 4 carbon atoms,wherein the carbon source can be in its simplest form as carbon dioxide or carbon monoxide. In particular, the carbon source may be any complex molecule with carbon in it. More in particular, the carbon source may be is selected from the group consisting of ethanol in combination with at least one other carbon source selected from the group consisting of acetate, propionate, butyrate, isobutyrate, valerate and hexanoate
and wherein the microorganism is selected from the group consisting of *Clostridium kluyveri* and *C. carboxidivorans.*

In a particular embodiment of the present invention, the microorganism expresses hydrogenase maturation protein and/or electron transport complex protein.

The microorganism according to any aspect of the present invention may be a genetically modified microorganism. The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. The wild type microorganism relative to the genetically modified microorganism of the present invention may have none or no detectable activity of the enzymes that enable the genetically modified microorganism to produce at least one carboxylic acid. As used herein, the term 'genetically modified microorganism' may be used interchangeably with the term 'genetically modified cell'. The genetic modification according to any aspect of the present invention is carried out on the cell of the microorganism.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell or microorganism. According to any aspect of the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more carboxylic acid and/or the respective carboxylic acid ester than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (carboxylic acid) in the nutrient medium.

In one example, the genetically modified microorganism according to any aspect of the present invention has increased expression relative to the wild type microorganism of hydrogenase maturation protein and/or electron transport complex protein. In particular, the hydrogenase maturation protein (hyd) may be selected from the group consisting of hydE, hydF or hydG. In particular, the hyd may comprise sequence identity of at least 50% to a polypeptide selected from the group consisting of CKL_0605, CKL_2330, CKL_3829 and the like. More in particular, the hyd used according to any aspect of the present invention may comprise a polypeptide with sequence identity of at least 50, 60, 65, 70, 75, 80, 85, 90, 91, 94, 95, 98 or 100% to a polypeptide selected from the group consisting of CKL_0605, CKL_2330 and CKL_3829.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that codes for a corresponding enzyme with increased activity and optionally by combining these measures. Genetically modified cells or microorganisms used in the method according to the invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extrachromosomally replicating vector.

The cells according to any aspect of the present invention are genetically transformed according to any method known in the art. In particular, the cells may be produced according to the method disclosed in WO/2009/077461.

The phrase 'the genetically modified cell has an increased activity, in comparison with its wild type, in enzymes' as used herein refers to the activity of the respective enzyme that is increased by a factor of at least 2, in particular of at least 10, more in particular of at least 100, yet more in particular of at least 1000 and even more in particular of at least 10000. In one example, the increased expression of an enzyme according to any aspect of the present invention may be 5, 10, 15, 20, 25, 30, 25, 40, 45, 50, 55, 60, 65, 70, 75, 80, 85, 90, 95 or 100% more relative to the expression of the enzyme in the wild type cell. In one example, the decreased expression of an enzyme according to any aspect of the present invention may be 5, 10, 15, 20, 25, 30, 25, 40, 45, 50, 55, 60, 65, 70, 75, 80, 85, 90, 95 or 100% less relative to the expression of the enzyme in the wild type cell.

In one example, the microorganism according to any aspect of the present invention may be capable of producing at least valeric acid and/or heptanoic acid from a carbon source of propionic acid and ethanol. In another example, the microorganism according to any aspect of the present invention may be capable of producing at least butyric acid from a carbon source of acetate and ethanol. In yet another example, the microorganism according to any aspect of the present invention may be capable of producing at least isohexanoic acid from a carbon source of isobutyric acid and ethanol. In one further example, the microorganism according to any aspect of the present invention may be capable of producing at least hexanoic acid from a carbon source of butyric acid and ethanol or a carbon source of acetate and ethanol.

Throughout this application, any data base code, unless specified to the contrary, refers to a sequence available from the NCBI data bases, more specifically the version online on 12 June 2014, and comprises, if such sequence is a nucleotide sequence, the polypeptide sequence obtained by translating the former.

The carboxylic acid may be any carboxylic known in the art. In particular, the carboxylic acid may be selected from the group consisting of Butanoic acid (CH₃(CH₂)₂COOH), Pentanoic acid (CH₃(CH₂)₃COOH), Hexanoic acid (CH₃(CH₂)₄COOH), Heptanoic acid (CH₃(CH₂)₅COOH, and Octanoic acid (CH₃(CH₂)₆COOH). In one example, the carboxylic acid may be selected from the group consisting of butyric, valeric, isovaleric, hexanoic, isohexanoic, heptanoic, isoheptanoic, octanoic and isooctanoic acid.

In one example, the carboxylic acid may be hexanoic acid.

The carboxylic acid is then subjected to Kolbe electrolysis, or to photo-Kolbe conditions. This reaction results in the formation of the respective alkane, carbon dioxide and hydrogen. The alkanes produced may be more immiscible. This allows for the alkane produced according to any aspect of the present invention to be easily separated. The method according to any aspect of the present invention may be considered to be highly selective and may allow for alkanes produced to have high purity. In one example, when hexanoic acid may be subjected to Kolbe electrolysis a decane (C₁₀H₂₂) and hydrogen, with minor amounts of pentane are formed. The hexane may be immiscible with the fermentation broth and may be easily separated.

Kolbe electrolysis conditions are known in the art, and include platinum electrodes, sono-emulsion with various electrodes such as boron-doped chemical vapour deposition diamond electrodes (Wadhawan J.D., et al., 2001), polymer electrodes, and the like. Kolbe electrolysis synonymous to Kolbe fatty acid or carboxylic acid electrolysis is a means of oxidative decarboxylation of carboxylic acids. Kolbe electrolysis typically works with the carboxylate anion rather than the acid itself.

In particular, the Kolbe electrolysis is an example of an organic redox reaction that takes place in an electrochemical cell. This method has several advantages which include for example the possibility to control the potential of the electrode. It may also be considered a simple reaction because no reducing or oxidizing agents are required. In Kolbe electrolysis, the oxidation does not take place chemically but electrolytically. Alkanes are formed by dimerisation of the generated radicals according to the reaction shown below.

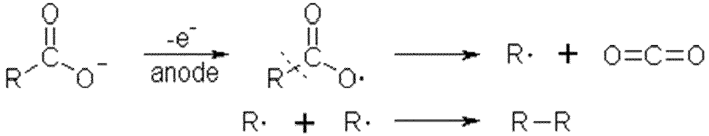

Kolbe electrolysis may also be known to produce side products which depend on the ease of the follow-up oxidation which leads to carbenium ions and their subsequent rearrangement:

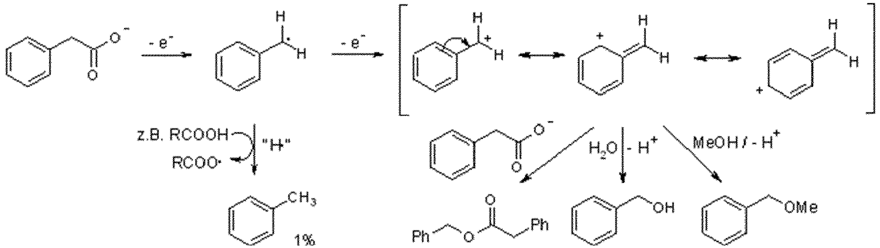

In one example, the Kolbe electrolysis is carried out on the fermentation broth itself that was used for carboxylic acid production in step (i) of the method according to any aspect of the present invention. This allows the alkane product to separate from the fermentation broth. The alkane may then be easily separated by decantation, distillation or other means known in the art. Thus, a continuous or semi-continuous process can be used, and the alkane may be removed, for example, using evaporative distillation, decantation, and the like from the fermentation broth. Both steps of (i) carboxylic acid production by the acetogenic bacteria and/or hydrogen oxidising bacteria and (ii) Kolbe electrolysis on the produced carboxylic acid may be carried out in a single fermenter and both steps may be carried out simultaneously. No separation of the carboxylic acid between steps (i) and (ii) may be needed.

According to any aspect of the present invention, the Kolbe electrolysis may comprise the presence of a salt, usually the alkali salt of the carboxylic acid, water and two metal electrodes. A current and voltage of at least 1V is available between the two electrodes.

In another example, the Kolbe electrolysis may be performed in methanol instead of water. Platinum electrodes may be used in this example. The use of methanol may be considered advantageous as it may yield excellent conversion rates and continuous electrolysis. Selectivity of the electrolysis may be improved with the use of methanol in Kolbe electrolysis.

In particular, Kolbe electrolysis may be performed in an electrolysis medium. The electrolysis medium may be water, methanol or a mixture of both. In one example, the mixture of both may mean the electrolysis medium comprises between about 0.5 percent to about 50 percent water by volume.

In another example, the fermentation is first completed, and then the carboxylic acid ions may be optionally isolated, before the Kolbe electrolysis is performed. The carboxylic acid can be removed from the fermentation broth, for example, by continuous extraction with a solvent. In this case, since the Kolbe electrolysis or photo-Kolbe electrolysis occurs after the fermentation takes place, the acetogenic bacteria can be collected, for example, by decantation or filtration of the fermentation media. The acetogenic bacteria may thus be recycled. The fermentation media including the carboxylic acid can be subjected to Kolbe electrolysis or photo-Kolbe electrolysis, and since the resulting products (respective alkane, by-product alkane, hydrogen and carbon dioxide) easily separate from the aqueous fermentation solution, they can be easily isolated, for example, by collecting the gases (by-product alkane, carbon dioxide and hydrogen) and separating them into their components, and by decanting or distilling the respective alkane. The aqueous solution can then be recycled to the fermenter, if desired, or otherwise disposed of. In this example, both steps may also be carried out in a single fermenter or separate fermenters.

In one example, one or more carboxylic acids may be subjected to the Kolbe electrolysis or photo-Kolbe electrolysis step at the same time. These carboxylic acids will form radicals of the alkyl group to which the carboxylic acid group is attached as shown above. These radicals can react with any alkyl radicals formed by the decarboxylation of carboxylic acid.

The carboxylic acid used according to any aspect of the present invention may be saturated, unsaturated and/or branched or unbranched. Accordingly, the resultant alkane may also be branched or unbranched depending on the carboxylic acid used. In particular, the resultant alkane may be symmetrical or unsymmetrical. A symmetrical alkane may be an alkane in which half of the molecule is a mirror image of the other half.

In particular, according to any aspect of the present invention, the alkane and carboxylic acid are selected from the group consisting of:
(a) the alkane comprising 6 carbon atoms and the carboxylic acid comprising 4 carbon atoms;
(b) the alkane comprising 8 carbon atoms and the carboxylic acid comprising 5 carbon atoms;
(c) the alkane comprising 10 carbon atoms and the carboxylic acid comprising 6 carbon atoms;
(d) the alkane comprising 12 carbon atoms and the carboxylic acid comprising 7 carbon atoms;
(e) the alkane comprising 14 carbon atoms and the carboxylic acid comprising 8 carbon atoms; and

More in particular, the alkane and carboxylic acid are selected from the group consisting of:
(c) the alkane comprising 10 carbon atoms and the carboxylic acid comprising 6 carbon atoms;
(d) the alkane comprising 12 carbon atoms and the carboxylic acid comprising 7 carbon atoms; and
(e) the alkane comprising 14 carbon atoms and the carboxylic acid comprising 8 carbon atoms;

In the method according to the present invention a combination of carboxylic acids may be produced comprising a first carboxylic acid and a second carboxylic acid and the alkane and the combination of carboxylic acids are selected from the group consisting of:
(f) the alkane comprises 8 carbon atoms and the first carboxylic acid comprises 6 carbon atoms and the second carboxylic acid comprises 4 carbon atoms;
(g) the alkane comprising 10 carbon atoms and the first carboxylic acid comprising 5 carbon atoms and the second carboxylic acid comprising 7 carbon atoms;
(h) the alkane comprising 12 carbon atoms and the first carboxylic acid comprising 8 carbon atoms and the second carboxylic acid comprising 6 carbon atoms;
(i) the alkane comprising 12 carbon atoms and the first carboxylic acid comprising 9 carbon atoms and the second carboxylic acid comprising 5 carbon atoms;
(j) the alkane comprising 14 carbon atoms and the first carboxylic acid comprising 6 carbon atoms and the second carboxylic acid comprising 8 carbon atoms;
(k)the alkane comprising 9 carbon atoms and the first carboxylic acid comprising 5 carbon atoms and the second carboxylic acid comprising 6 carbon atoms;
(I) the alkane comprising 11 carbon atoms and the first carboxylic acid comprising 6 carbon atoms and the second carboxylic acid comprising 7 carbon atoms; and
(m) the alkane comprising 13 carbon atoms and the first carboxylic acid comprising 7 carbon atoms and the second carboxylic acid comprising 8 carbon atoms.

The carboxylic acid used in any aspect of the present invention may comprise at least 4 carbon atoms (butanoic acid/butyric acid). When butyric acid is subjected to Kolbe electrolysis, the resultant alkane may comprise at least 6 carbon atoms. In particular, the resultant alkane may be a hexane. In another example, when a combination of carboxylic acids comprising at least a first carboxylic acid of butyric acid and a second carboxylic acid of propionic acid (3 carbon atoms) the resultant alkane may comprise at least 5 carbon atoms. In particular, the resultant alkane may be a pentane. By-products such as hexane and butane may also be formed in this example.

In one example, the carboxylic acid used in any aspect of the present invention may comprise at least 5 carbon atoms (pentanoic acid/ valeric acid). When pentanoic acid is subjected to Kolbe electrolysis, the resultant alkane may comprise at least 8 carbon atoms. In particular, the resultant alkane may be an octane. In a further example, when a combination of carboxylic acids comprising at least a first carboxylic acid of pentanoic acid and a second carboxylic acid of propionic acid the resultant alkane may comprise at least 6 carbon atoms. In particular, the resultant alkane may be a hexane. By-products such as octane and butane may also be formed in this example. In yet another example, when a combination of carboxylic acids comprising at least a first carboxylic acid of pentanoic acid and a second carboxylic acid of butanoic acid the resultant alkane may comprise at least 7 carbon atoms. In particular, the resultant alkane may be a heptane. By-products such as octane and hexane may also be formed in this example. In one example, the carboxylic acid used in any aspect of the present invention may comprise at least 6 carbon atoms (hexanoic acid/ caproic acid). When hexanoic acid is subjected to Kolbe electrolysis, the resultant alkane may comprise at least 10 carbon atoms. In particular, the resultant alkane may be a decane. In another example, when a combination of carboxylic acids comprising at least a first carboxylic acid of hexanoic acid and a second carboxylic acid of propionic acid the resultant alkane may comprise at least 7 carbon atoms. In particular, the resultant alkane may be a heptane. By-products such as decane and butane may also be formed in this example. In another example, when a combination of carboxylic acids comprising at least a first carboxylic acid of hexanoic acid and a second carboxylic acid of butanoic acid the resultant alkane may comprise at least 8 carbon atoms. In particular, the resultant alkane may be an octane. By-products such as decane and hexane may also be formed in this example. In yet another example, when a combination of carboxylic acids comprising at least a first carboxylic acid of hexanoic acid and a second carboxylic acid of pentanoic acid, the resultant alkane may comprise at least 9 carbon atoms. In particular, the resultant alkane may be a nonane. By-products such as decane and octane may also be formed in this example.

In one example, the carboxylic acid used in any aspect of the present invention may comprise at least 7 carbon atoms (heptanoic acid/ enanthic acid). When heptanoic acid is subjected to Kolbe electrolysis, the resultant alkane may comprise at least 12 carbon atoms. In particular, the resultant alkane may be a dodecane. In another example, when a combination of carboxylic acids comprising at least a first carboxylic acid of heptanoic acid and a second carboxylic acid of propionic acid the resultant alkane may comprise at least 8 carbon atoms. In particular, the resultant alkane may be an octane. By-products such as dodecane and butane may also be formed in this example. In another example, when a combination of carboxylic acids comprising at least a first carboxylic acid of heptanoic acid and a second carboxylic acid of butanoic acid the resultant alkane may comprise at least 9 carbon atoms. In particular, the resultant alkane may be a nonane. By-products such as dodecane and hexane may also be formed in this example. In yet another example, when a combination of carboxylic acids comprising at least a first carboxylic acid of heptanoic acid and a second carboxylic acid of pentanoic acid, the resultant alkane may comprise at least 10 carbon atoms. In particular, the resultant alkane may be a decane. By-products such as dodecane and octane may also be formed in this example. In a further example, when a combination of carboxylic acids comprising at least a first carboxylic acid of heptanoic acid and a second carboxylic acid of hexanoic acid, the resultant alkane may comprise at least 11 carbon atoms. In particular, the resultant alkane may be an undecane. By-products such as dodecane and decane may also be formed in this example.

In one example, the carboxylic acid used in any aspect of the present invention may comprise at least 8 carbon atoms (octanoic acid/ caprylic acid). When octanoic acid is subjected to Kolbe electrolysis, the resultant alkane may comprise at least 14 carbon atoms. In particular, the resultant alkane may be a tetradecane. In another example, when a combination of carboxylic acids comprising at least a first carboxylic acid of octanoic acid and a second carboxylic acid of propionic acid the resultant alkane may comprise at least 9 carbon atoms. In particular, the resultant alkane may be a nonane. By-products such as tetradecane and butane may also be formed in this example. In another example, when a combination of carboxylic acids comprising at least a first carboxylic acid of octanoic acid and a second carboxylic acid of butanoic acid the resultant alkane may comprise at least 10 carbon atoms. In particular, the resultant alkane may be a decane. By-products such as tetradecane and hexane may also be formed in this example. In yet another example, when a combination of carboxylic acids comprising at least a first carboxylic acid of octanoic acid and a second carboxylic acid of pentanoic acid, the resultant alkane may comprise at least 11 carbon atoms. In particular, the resultant alkane may be an undecane. By-products such as tetradecane and octane may also be formed in this example. In a further example, when a combination of carboxylic acids comprising at least a first carboxylic acid of octanoic acid and a second carboxylic acid of hexanoic acid, the resultant alkane may comprise at least 12 carbon atoms. In particular, the resultant alkane may be a dodecane. By-products such as tetradecane and decane may also be formed in this example. In yet another example, when a combination of carboxylic acids comprising at least a first carboxylic acid of octanoic acid and a second carboxylic acid of heptanoic acid, the resultant alkane may comprise at least 13 carbon atoms. In particular, the resultant alkane may be a tridecane. By-products such as tetradecane and dodecane may also be formed in this example.

The carboxylic acid used in any aspect of the present invention comprises at least 6, 7, or 14 carbon atoms. When the acid is subjected to Kolbe electrolysis, the resultant alkane may comprise at least 10, 12 or 14 carbon atoms respectively. A combination of carboxylic acids may be used in any aspect of the present invention. There may be 2, 3, 4, 5, 6, 7, 8, 9, 10 or more carboxylic acids subjected to Kolbe electrolysis. A mixture of alkanes may be formed depending on which first acid dimerises with which second acid. A skilled person would be able to select the carboxylic acids to undergo Kolbe electrolysis to produce the desired alkane(s).

The method according to any aspect of the present invention is advantageous as it provides hydrocarbons in the gasoline range, from the same starting materials as those used to form ethanol, but which have higher energy per unit volume.

The microorganisms according to any aspect of the present invention may be held in immobilized cell bioreactors, such as fibrous-bed bioreactors (FBBs), to carry out the fermentation. However, any known fermenter may be used. A skilled person would easily be able to determine the most suitable fermenter to use based on the method used to obtain carboxylic acid, the type of carboxylic acids produced, the conditions for growth of the microorganisms and the like.

The use of immobilized cell bioreactors helps re-use the bacteria. However, in examples where Kolbe electrolysis and/or photo-Kolbe electrolysis is carried out on the fermentation media, the alkane will be easily removed from the reaction media, so immobilization of the bacteria will not be a problem in such cases. When the bacteria are not immobilized, they can be isolated, for example, using centrifugal bacterial reclamation. Continuous decantation, evaporative removal, or extraction of the alkane allows the fermentation medium to be continuously sterilized and minimizes water use. The non-stop nature of the fermentation process allows substrate concentrations to be constantly kept at optimal levels and therefore fermentation efficiency is maximized.

The carboxylic acid(s) formed during the fermentation can be converted to alkane via Kolbe electrolysis. Kolbe electrolysis is an anodic oxidation process of a carboxylate anion. A radical is formed, which then decarboxylates. The resulting radical combines with another to form a dimer. For example, the carboxylic acid formed according to any aspect of the present invention may lose a mole of carbon dioxide to produce a alkyl radical, two of which will combine to form the respect alkane. The efficiency of Kolbe electrolysis is sensitive to water. Therefore, in one example, the reaction may be run in (almost) water free conditions.

In one example, the Kolbe electrolysis may be performed in an ionic liquid, which can optionally be present in the fermenter. In another example, the anion exchange membranes are used as solid polymer electrolytes (http://www.pca-gmbh.com/appli/spe.htm). One example of a suitable electrode system is a monel cathode and platinum sheet or platinum gauze anode.

In Kolbe electrolysis, there are some competing reactions, such as an elimination reaction which would convert the carboxylic acid to alkene, carbon dioxide and/or hydrogen. Low temperatures (i.e., 30-40°C) and high flow rates over the electrodes tend to favour paraffin formation, rather than elimination.

The alkane according to any aspect of the present invention may be used to produce at least one oxidised alkane product, wherein the method comprises contacting the alkane according to any aspect of the present invention with at least one second microorganism capable of oxidising the alkane to the respective oxidised alkane product, wherein the oxidised alkane product is selected from the group consisting of respective alcohols, carboxylic acids and dicarboxylic acids and the second microorganism is selected from the group consisting of *E. coli, Candida tropicalis, Yarrowia lipolytica,* and *Pseudomonas putida.*

In one example, the microorganism according to any aspect of the present invention may be further genetically modified to be capable of oxidising the alkane to any one of the respective oxidised products. In another example, the alkane produced according to any aspect of the present invention is fed to a second microorganism that may be capable of carrying out the oxidation of the alkane.

The second microorganism according to any aspect of the present invention may be selected from the group consisting of *E. coli, Candida tropicalis, Yarrowia lipolytica, Pseudomonas putida* and the like. In particular, the second microorganism may be genetically modified to increase the expression of at least one enzyme that may be capable of oxidising the alkane. In one example, the second microorganism may be genetically modified to express a recombinant alkane hydroxylase. In particular, the alkane hydroxylase may be a cytochrome P450 monooxygenase of the CYP153 family. The term "cytochrome P450 monooxygenase of the CYP153 family" may refer herein to a cytosolic oxidase which is part of a 3-component system comprising furthermore a ferredoxin and a ferredoxin reductase, with an alkane-binding site and the ability to hydroxylate alkanes. More in particular, the cytochrome P450 monooxygenase of the CYP153 family may have at least 80, 90, 95 or 99 % polypeptide sequence identity with cytochrome P450 monooxygenase of the CYP153 family from *Alcanivorax borkumensis* SK2 (database code YP_691921). The second microorganism may further have alkane hydroxylase activity.

In particular, the term "alkane hydroxylase activity", as used herein, refers to the ability to catalyse the hydroxylation of alkanes or unsubstituted linear alkyl radicals comprising at least six, more in particular at least twelve, carbon radicals. The term "cytochrome P450 monooxygenase of the CYP153 family" means a non-membrane-bound oxidase which comprises a binding site for alkanes, unsubstituted linear alkyl radicals comprising at least five, or particularly twelve, carbon radicals or monohydroxylated alkanes, and the polypeptide chain of which comprises the motif LL(I/L)(V/I)GGNDTTRN. In one example, a "cytochrome P450 monooxygenase of the CYP153 family", as used herein, is a cytochrome P450 monooxygenase of the CYP153 family from *Alcanivorax borkumensis* SK2 (database code YP_691921) or a variant which preferably has alkane hydroxylase activity.

The enzymes used according to any aspect of the present the invention may be recombinant enzymes. The term "recombinant", as used herein, refers to the possibility that the corresponding nucleic acid molecule may not be present in the natural cell and/or was produced using genetic engineering methods. In one example, a protein is said to be recombinant if the corresponding polypeptide is encoded by a recombinant nucleic acid. Similarly, a recombinant cell, as used herein, refers to a cell which has at least one recombinant nucleic acid or one recombinant polypeptide. A person skilled in the art is familiar with processes suitable for producing recombinant molecules or cells. Recombinant enzymes may be overexpressed, for example by using pET- or pGEX-vector systems which are known to a person skilled in the art.

In one example, to supply cytochrome P450 monooxygenase of the CYP153 family with electrons from the reducing agent, preferably NADH, in an optimal way, the cell expressing the monooxygenase together with ferredoxin reductase and ferredoxin, both of which interact functionally with said monooxygenase may be used. The polypeptides may be isolated or, when using a whole cell catalyst, co-expressed polypeptides or polypeptides fused N- or C-terminally to the cytochrome P450 monooxygenase of the CYP153 family. A person skilled in the art can readily determine whether a ferredoxin reductase or a ferredoxin interacts functionally with a given cytochrome P450 monooxygenase of the CYP153 family by whether the reducing agent is oxidized in the presence of an alkane substrate and the three polypeptides. Alternatively, the enzyme assay described by Scheps, D. et al. (2011) may be used, which shows a distinct increase in the reaction rate in the case of functionally interacting polypeptides. In one example, cytochrome P450 monooxygenase of the CYP153 family, ferredoxin and ferredoxin reductase are from the same organism. In another example, ferredoxin reductase may be that from *Alcanivorax borkumensis* SK2 (database code YP_691923) or may be a variant thereof, ferredoxin may be that from *Alcanivorax borkumensis* SK2 (database code YP_691920) or is a variant thereof, and cytochrome P450 monooxygenase of the CYP153 family may be that from *Alcanivorax borkumensis* SK2 (database code YP_691921) or is a variant thereof.

In another example, the alkane hydroxylase may be an AlkB monooxygenase. AlkB is an oxidoreductase first known from the *Pseudomonas putida* Gpo1 AlkBGT system, which is dependent on another two polypeptides, AlkG and AlkT. AlkT is characterized as FAD-dependent rubredoxin reductase that passes on electrons from NADH to AlkG. AlkG is a rubredoxin, an iron-containing redox protein that acts as a direct electron donor for AlkB. The term "AlkB monooxygenase" used herein may refer to a polypeptide with a sequence homology of at least, in the order of increasing preference, 75, 80, 85, 90, 92, 94, 96, 98 or 99% to the sequence of *Pseudomonas putida* Gpo1 AlkB (database code: CAB54050.1), which polypeptide is capable of oxidizing alkanes. In one example, the AlkB monooxygenase may be an alkane-oxidizing oxidoreductase which functionally acts together with the *Pseudomonas putida* Gpo1 AlkG (CAB54052.1) and AlkT (CAB54063.1) polypeptides. For optimal supply of AlkB alkane hydroxylase with electrons, the cell expressing the monooxygenase together with auxiliary proteins that functionally interact with it, particularly AlkG and/or AlkT or respective variants thereof, from *Pseudomonas putida* Gpo1 AlkG (CAB54052.1) and AlkT (CAB54063.1) polypeptides may be used.

The ability of the second microorganism used in the process of the invention to oxidize substrates may be enhanced by the second microorganism expressing an alcohol dehydrogenase as an alternative to or additionally to the alkane hydroxylase. In particular, the term "alcohol dehydrogenase", as used herein, refers to an enzyme that oxidizes an aldehyde or ketone to the corresponding primary or secondary alcohol. Examples include the alcohol dehydrogenases of *Ralstonia eutropha* (ACB78191.1), *Lactobacillus brevis* (YP_795183.1), *Lactobacillus kefiri* (ACF95832.1), from horse liver, of *Paracoccus pantotrophus* (ACB78182.1) and *Sphingobium yanoikuyae* (EU427523.1), and also the respective variants thereof. The alkanes produced according to any aspect of the present invention may thus be readily used as a carbon source for the second microorganism to produce at least one respect oxidised alkane product. This method may allow for a purer source of alkane to be used to produce the oxidised products thus resulting in a more specific process of producing oxidised alkane products and less by-products being formed.

The method according to any aspect of the present invention may comprise a further step of isolating the alkane. For example, the alkane produced according to any aspect of the present invention may be of a different phase from the reaction mixture. Isolation of the alkanes may thus be by simple means of separation known to a skilled person, for example, centrifugation and then decanting. In one example, at least one olefin is produced after Kolbe electrolysis of the carboxylic acid. In another example, the alkane is further subjected to catalytic reforming and/or isomerization to form gasoline or components of a gasoline composition.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Clostridium kluyveri forming butyric acid from acetate and ethanol

For the biotransformation of ethanol and acetate to butyric acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCI (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂0, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 27 h to an OD₆₀₀ₙₘ >0.6. Then the cell suspension was centrifuged, washed with production buffer (pH 6.0; 8.32 g/L K-acetate, 0.5 g/l ethanol) and centrifuged again.

For the production culture, 200 ml of production buffer in a 500 ml bottle was inoculated with the washed cells from the main culture to an OD₆₀₀ₙₘ of 0.2. The culture was capped with a butyl rubber stopper and incubated for 71 h at 37°C and 100 rpm in an open water shaking bath. At the start and end of the culturing period, samples were taken. These were tested for optical density, pH and the different analytes (tested by NMR).

The results showed that in the production phase the amount of acetate decreased from 5.5 g/l to 5.0 g/l and the amount of ethanol decreased from 0.5 g/l to 0.0 g/l. Also, the concentration of butyric acid was increased from 0.05 g/l to 0.8 g/l and the concentration of hexanoic acid was increased from 0.005 g/l to 0.1 g/l.

### Example 2

### Clostridium kluyveri forming hexanoic acid from acetate and ethanol

For the biotransformation of ethanol and acetate to hexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCI (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 27 h to an OD₆₀₀ₙₘ >0.6. Then the cell suspension was centrifuged, washed with production buffer (pH 6.0; 0.832 g/L K-acetate, 5.0 g/l ethanol) and centrifuged again.

For the production culture, 200 ml of production buffer in a 500 ml bottle was inoculated with the washed cells from the main culture to an OD₆₀₀ₙₘ of 0.2. The culture was capped with a butyl rubber stopper and incubated for 71 h at 37°C and 100 rpm in an open water shaking bath. At the start and end of the culturing period, samples were taken. These were tested for optical density, pH and the different analytes (tested by NMR).

The results showed that in the production phase the amount of acetate decreased from 0.54 g/l to 0.03 g/l and the amount of ethanol decreased from 5.6 g/l to 4.9 g/l. Also, the concentration of butyric acid was increased from 0.05 g/l to 0.28 g/l and the concentration of hexanoic acid was increased from 0.03 g/l to 0.79 g/l.

### Example 3

### Clostridium kluyveri forming hexanoic acid from butyric acid and ethanol

For the biotransformation of ethanol and butyric acid to hexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCI (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂0, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.3.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 25 h to an OD₆₀₀ₙₘ >0.4. Then the cell suspension was centrifuged, washed with production buffer (pH 6.16; 4.16 g/L K-acetate, 10.0 g/l ethanol) and centrifuged again.

For the production cultures, 200 ml of production buffer in a 500 ml bottle was inoculated with the washed cells from the main culture to an OD₆₀₀ₙₘ of 0.2. In a first culture, at the beginning 1.0 g/l butyric acid was added to the production buffer, in a second culture, no butyric acid was added to the production buffer. The cultures were capped with a butyl rubber stopper and incubated for 71h at 37°C and 100 rpm in an open water shaking bath. At the start and end of the culturing period, samples were taken. These were tested for optical density, pH and the different analytes (tested by NMR).

The results showed that in the production phase of the butyric acid supplemented culture the amount of acetate decreased from 3.1 g/l to 1.1 g/l and the amount of ethanol decreased from 10.6 g/l to 7.5 g/l. Also, the concentration of butyric acid was increased from 1.2 g/l to 2.2 g/l and the concentration of hexanoic acid was increased from 0.04 g/l to 2.30 g/l.

In the production phase of the non-supplemented culture the amount of acetate decreased from 3.0 g/l to 1.3 g/l and the amount of ethanol decreased from 10.2 g/l to 8.2 g/l. Also, the concentration of butyric acid was increased from 0.1 g/l to 1.7 g/l and the concentration of hexanoic acid was increased from 0.01 g/l to 1.40 g/l.

### Example 4

### Clostridium kluyveri forming isohexanoic acid from isobutyric acid and ethanol

For the biotransformation of ethanol and isobutyric acid to isohexanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCI (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 144 h to an OD₆₀₀ₙₘ >0.3.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 25 h to an OD₆₀₀ₙₘ >0.4. Then the cell suspension was centrifuged, washed with production buffer (pH 6.16; 4.16 g/L K-acetate, 10.0 g/l ethanol) and centrifuged again.

For the production culture, 200 ml of production buffer in a 500 ml bottle was inoculated with the washed cells from the main culture to an OD₆₀₀ₙₘ of 0.2. At the beginning 1.0 g/l isobutyric acid was added to the production buffer. The culture was capped with a butyl rubber stopper and incubated for 71h at 37°C and 100 rpm in an open water shaking bath. At the start and end of the culturing period, samples were taken. These were tested for optical density, pH and the different analytes (tested by NMR).

The results showed that in the production phase the amount of acetate decreased from 3.7 g/l to 1.0 g/l, the amount of ethanol decreased from 12.7 g/l to 7.8 g/l and the amount of isobutyric acid decreased from 1.30 g/l to 0.98 g/l. Also, the concentration of butyric acid was increased from 0.1 g/l to 1.6 g/l, the concentration of hexanoic acid was increased from 0.02 g/l to 1.80 g/l and the concentration of isohexanoic acid was increased from 0.00 g/l to 0.07 g/l.

### Example 5

### Clostridium kluyveri forming valeric acid and heptanoic acid from propionic acid and ethanol

For the biotransformation of ethanol and propionic acid to valeric acid and heptanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCI (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 119 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 21 h to an OD₆₀₀ₙₘ >0.4. Then the cell suspension was centrifuged, washed with production buffer (pH 6.0; 1.0 g/L propionic acid, 2.5 g/l ethanol) and centrifuged again.

For the production culture, 200 ml of production buffer in a 500 ml bottle was inoculated with the washed cells from the main culture to an OD₆₀₀ₙₘ of 0.2. The culture was capped with a butyl rubber stopper and incubated for 68 h at 37°C and 100 rpm in an open water shaking bath. At the start and end of the culturing period, samples were taken. These were tested for optical density, pH and the different analytes (tested by NMR).

The results showed that in the production phase the amount of propionic acid decreased from 1.08 g/l to 0.04 g/l and the amount of ethanol decreased from 2.5 g/l to 1.5 g/l. Also, the concentration of valeric acid was increased from 0.05 g/lto 0.95 g/l and the concentration of heptanoic acid was increased from 0.00 g/l to 0.29 g/l.

### Example 6

### Kolbe electrolysis of hexanoic acid in water.

The Kolbe-electrolysis of 1M hexanoic acid with 0.5 eq KOH was carried out in water with platinum electrodes. A 3-necked-flask was connected to a cooling trap where the developed gases were condensed at -80°C. The aim of the experiment was to obtain a mass-balance as accurate as possible. At the beginning of the transformation two layers could be observed, since hexanoic acid is not totally soluble in water at c = 1M. During the process the reaction mixture turned milky (almost only one layer), and at the end again two layers could be observed again. All three phases, the condensed phase with volatile products, the aqueous layer and the organic layer of the reaction mixture, were analysed with GC-MS and ¹H-NMR. The parameters and results of the electrolysis are listed in Table 1.

**Table 1. Kolbe electrolysis of hexanoic acid in water.**

| | |
|---|---|
| C ᵢₙᵢₜ [M] | 1.0 |
| conversion rate | 80% |
| pH | 5.8 - 9.0 |
| current density [mA/cm²] | 300 |
| cell voltage _{init-final} [V] | 7.9 - 5.3 |
| aqueous layer | unreacted hexanoic acid 20% |
| volatile fraction* | 1-pentene 14%, 2-pentene 7%, pentane 5% |
| organic layer | decane 39 % |
| not determined | 20% (e.g. volatile gases, esters, aldehydes) |

| | |
|---|---|
| ** The volatile fraction was condensed and trapped in CDCl₃ or C₆D₆* | |

### Example 7

### Kolbe electrolysis of hexanoic acid in water. Dependency with the concentration of starting material.

A row of experiments with hexanoic acid in water at different concentrations was carried out. The reaction vessel was connected again to a cooling trap in order to collect the volatile compounds. Once the reaction was finished, the aqueous layer was analysed by ¹H-NMR, the layers were then separated and analysed (in case of reactions at high concentrations) or it was divided into two: one half extracted with pentane for GC-MS analysis, and the other half extracted with CDCl₃ or C₆D₆ for ¹H-NMR analysis. The results are shown in Table 2 below. All the experiments were carried out with hexanoic acid containing 0.5 eq of KOH (pH∼5) using platinum electrodes. At initial concentrations of hexanoic acid c < 1M, the Kolbe-electrolysis was not selective anymore, and new products from competitive processes became more important. The formation of aldehydes and esters was observed at all concentrations.

**Table 2. Kolbe electrolysis of hexanoic acid in water of varying concentration.**

| entry | 1 | 2 | 3 | 4 |
|---|---|---|---|---|
| c ᵢₙᵢₜ [M] | 1.0 | 0.5 | 0.1 | 0.05 |
| conversion | 83% | 84% | 82% | 82% |
| pH | 5.8 - 9.0 | 5.4 - 9.4 | 5.2 - 7.5 | 5.0 - 7.1 |
| current density [mA/cm²] | 300 | 300 | 100 | 100 |
| voltage _{init - final} [V] | 7.9 - 5.3 | 14.0 - 8.4 | 8.3 - 8.0 | 11.5 - 14.8 |
| volatile fraction* | 1-pentene 44% | 1-pentene 44% | 1-pentene 44% | 1-pentene 38% |
| | 2-penten 25% | 2-pentene 26% | 2-pentene 26% | 2-pentene 34% |
| | pentane 31 % | pentane 30% | pentane 30% | pentane 28% |
| organic layer | decane 90 % | decane 82% | decane 0% | decane 0% |

| | | | | |
|---|---|---|---|---|
| ** The volatile fraction was condensed and trapped in CDCl₃ or C₆D₆* | | | | |

### Example 8

### Kolbe electrolysis in water of heptanoic acid

The Kolbe-electrolysis of 1M heptanoic acid containing 0.5eq KOH was carried out in water with platinum electrolyses in a 3-necked-flask. The aim of the experiment was to obtain a mass-balance as accurate as possible. At the beginning of the electrolysis two layers could be observed, since heptanoic acid is not totally soluble in water at c = 1M. During the process the reaction mixture turned milky (almost only one layer), and at the end again two layers could be observed again. After a conversion of about 90%, the electrolysis was stopped. Yield of the separated organic layer was 79% as shown in Table 3. The aqueous layer and the organic layer, were analysed with GC-MS and 1H-NMR. The organic layer had mainly dodecane and octane, and other not determined alkanes and traces of alkenes. The aqueous layer consisted mainly of unreacted heptanoic acid and traces of shorter carboxylic acids (e.g. hexanoic acid), alcohols (e.g. pentanole) and esters, that can be explained as Kolbe-products as well.

**Table 3. Kolbe electrolysis of heptanoic acid in water.**

| | |
|---|---|
| **C** ᵢₙᵢₜ [M] | 1 |
| conversion rate | 92% |
| pH | 5.5 - 9.0 |
| current density [mA/cm²] | 1000 |
| cell voltage _{init-final} [V] | 30 - 11 |
| aqueous layer* | unreacted acid 8% |
| organic layer | 79% alkanes (thereof: dodecane 42%) |
| *** not determined | e.g. short chain fatty acids, alcohols, esters... |

### Example 9

### Kolbe electrolysis in methanol of hexanoic acid.

The Kolbe-electrolysis of 1M hexanoic acid with 0.5 eq NaOMe was carried out in methanol with platinum electrodes at high current densities. These conditions led to a passivation of the electrodes in a few minutes and end up in a slowdown of the electrolysis. In order to avoid any passivation of the electrodes an automatic polarity reversal of the electrodes was used to yield excellent conversion rates and a continuous electrolysis. This kind of processing led to a high decane selectivity in methanol. This example showed that the selectivity of the electrolysis in methanol is much higher than in water under the same conditions. Once the reaction was finished, the electrolyte was analysed by ¹H-NMR and the results shown in Table 4.

**Table 4. Kolbe electrolysis of heptanoic acid in methanol.**

| | |
|---|---|
| C ᵢₙᵢₜ [M] | 1.0 |
| conversion rate | 50 % |
| current density [mA/cm²] | 1000 |
| cell voltage _{init-final} [V] | 25 - 35 |
| selectivity | 95% decane, < 5% others (e.g. formic acid) |
| traces | volatile gases, esters, aldehydes |

### References

Wadhawan J.D., et al., 2001, Pure and Applied Chemistry, 73(12):1947-1955, Steinbusch, 2011, Zhang, 2013, Van Eerten-Jansen, M. C. A. A, 2013, Ding H. et al, 2010, Barker H.A., 1949, Stadtman E.R., 1950, Bornstein B. T., et al., 1948, Byoung, S.J et al. 2013, Seedorf, H., et al., 2008, PNAS 105 (6): 2128-2133, 'Extraction Model' in Kieun C., et al., 2013, Mat-Jan et al., Bunch et al., 1997, Richard J. Heath et al., 1995, Chang et al., 1983, Abdel-Ahmid et al., 2001, Reed, 1981, Drake et al., 2006, Drake & Kusel, 2005, Wood, 1991, Drake et al., 2004, Scheps, D., Malca, H., Hoffmann, B., Nestl, B. M, and Hauer, B. (2011) Org. Biomol. Chem., 9, 6727, US20140051136, WO/2009/077461, WO 98/00558, WO 00/68407, WO2007/0275447 US2008/0057554

## Claims

1. A method of producing at least one alkane, the method comprising,
- producing at least one carboxylic acid from a carbon source using at least one microorganism, and
- performing Kolbe electrolysis on the carboxylic acid to produce the alkane,
wherein the alkane comprises at least 6 carbon atoms and the carboxylic acid comprises at least 4 carbon atoms, wherein the carbon source is selected from the group consisting of ethanol in combination with at least one other carbon source selected from the group consisting of acetate, propionate, butyrate, isobutyrate, valerate and hexanoate and wherein the microorganism is selected from the group consisting of *Clostridium kluyveri* and C. *carboxidivorans.*

2. The method according to claim 1, wherein the microorganism expresses hydrogenase maturation protein and/or electron transport complex protein.

3. The method according to either claim 1 or 2, wherein the microorganism is genetically modified and the genetically modified microorganism has increased expression relative to the wild type microorganism of at least one enzyme selected from the group consisting of hydrogenase maturation protein and/or electron transport complex protein.

4. The method according to any of the preceding claims, wherein the alkane is branched or unbranched.

5. The method according to any one of the preceding claims, wherein the alkane and carboxylic acid are selected from the group consisting of:
(a) the alkane comprising 6 carbon atoms and the carboxylic acid comprising 4 carbon atoms;
(b) the alkane comprising 8 carbon atoms and the carboxylic acid comprising 5 carbon atoms;
(c) the alkane comprising 10 carbon atoms and the carboxylic acid comprising 6 carbon atoms;
(d) the alkane comprising 12 carbon atoms and the carboxylic acid comprising 7 carbon atoms; and
(e) the alkane comprising 14 carbon atoms and the carboxylic acid comprising 8 carbon atoms.

6. The method according to any one of the preceding claims, wherein the alkane and carboxylic acid are selected from the group consisting of:
(c) the alkane comprising 10 carbon atoms and the carboxylic acid comprising 6 carbon atoms;
(d) the alkane comprising 12 carbon atoms and the carboxylic acid comprising 7 carbon atoms; and
(e) the alkane comprising 14 carbon atoms and the carboxylic acid comprising 8 carbon atoms.

7. The method according to any one of claims 1 to 3, wherein a combination of carboxylic acids is produced comprising a first carboxylic acid and a second carboxylic acid and the alkane and the combination of carboxylic acids are selected from the group consisting of:
(f) the alkane comprises 8 carbon atoms and the first carboxylic acid comprises 6 carbon atoms and the second carboxylic acid comprises 4 carbon atoms;
(g) the alkane comprising 10 carbon atoms and the first carboxylic acid comprising 5 carbon atoms and the second carboxylic acid comprising 7 carbon atoms;
(h) the alkane comprising 12 carbon atoms and the first carboxylic acid comprising 8 carbon atoms and the second carboxylic acid comprising 6 carbon atoms;
(i) the alkane comprising 12 carbon atoms and the first carboxylic acid comprising 9 carbon atoms and the second carboxylic acid comprising 5 carbon atoms;
(j) the alkane comprising 14 carbon atoms and the first carboxylic acid comprising 6 carbon atoms and the second carboxylic acid comprising 8 carbon atoms;
(k) the alkane comprising 9 carbon atoms and the first carboxylic acid comprising 5 carbon atoms and the second carboxylic acid comprising 6 carbon atoms;
(I) the alkane comprising 11 carbon atoms and the first carboxylic acid comprising 6 carbon atoms and the second carboxylic acid comprising 7 carbon atoms; and
(m) the alkane comprising 13 carbon atoms and the first carboxylic acid comprising 7 carbon atoms and the second carboxylic acid comprising 8 carbon atoms.

8. The method according to any of the preceding claims, wherein the method comprises a further step of isolating the alkane.

9. The method according to any one of the preceding claims, wherein the Kolbe electrolysis is performed in an electrolysis medium comprising methanol.

10. A method of producing at least one oxidised alkane product, wherein the method comprises producing an alkane according to the method of any one of the claims 1 to 9 and subsequently contacting the alkane with at least one second microorganism capable of oxidising the alkane to the respective oxidised alkane product, wherein the oxidised alkane product is selected from the group consisting of respective alcohols, carboxylic acids and dicarboxylic acids and the second microorganism is selected from the group consisting of *E. coli, Candida tropicalis, Yarrowia lipolytica* and *Pseudomonas putida.*

## Patentansprüche

1. Verfahren zur Herstellung wenigstens eines Alkans, wobei das Verfahren
- Herstellen wenigstens einer Carbonsäure aus einer Kohlenstoffquelle unter Verwendung wenigstens eines Mikroorganismus und
- Durchführen einer Kolbe-Elektrolyse an der Carbonsäure zur Herstellung des Alkans
umfasst, wobei das Alkan wenigstens 6 Kohlenstoffatome und die Carbonsäure wenigstens 4 Kohlenstoffatome umfasst, wobei die Kohlenstoffquelle ausgewählt ist aus der Gruppe bestehend aus Ethanol in Kombination mit wenigstens einer anderen Kohlenstoffquelle ausgewählt aus der Gruppe bestehend aus Acetat, Propionat, Butyrat, Isobutyrat, Valerat und Hexanoat und wobei der Mikroorganismus ausgewählt ist aus der Gruppe bestehend aus *Clostridium kluyveri* und *C. carboxidivorans.*

2. Verfahren nach Anspruch 1, wobei der Mikroorganismus Hydrogenase-Reifungsprotein und/oder Elektronentransportkomplex-Protein exprimiert.

3. Verfahren nach Anspruch entweder Anspruch 1 oder 2, wobei der Mikroorganismus gentechnisch verändert ist und der gentechnisch veränderte Mikroorganismus relativ zum Wildtyp-Mikroorganismus eine erhöhte Expression wenigstens eines Enzyms ausgewählt aus der Gruppe bestehend aus Hydrogenase-Reifungsprotein und/oder Elektronentransportkomplex-Protein aufweist.

4. Verfahren nach einem der vorhergehenden Ansprüche, wobei das Alkan verzweigt oder unverzweigt ist.

5. Verfahren nach einem der vorhergehenden Ansprüche, wobei das Alkan bzw. die Carbonsäure ausgewählt sind aus der Gruppe bestehend aus:
(a) dem Alkan, das 6 Kohlenstoffatome umfasst, und der Carbonsäure, die 4 Kohlenstoffatome umfasst;
(b) dem Alkan, das 8 Kohlenstoffatome umfasst, und der Carbonsäure, die 5 Kohlenstoffatome umfasst;
(c) dem Alkan, das 10 Kohlenstoffatome umfasst, und der Carbonsäure, die 6 Kohlenstoffatome umfasst;
(d) dem Alkan, das 12 Kohlenstoffatome umfasst, und der Carbonsäure, die 7 Kohlenstoffatome umfasst; und
(e) dem Alkan, das 14 Kohlenstoffatome umfasst, und der Carbonsäure, die 8 Kohlenstoffatome umfasst.

6. Verfahren nach einem der vorhergehenden Ansprüche, wobei das Alkan bzw. die Carbonsäure ausgewählt sind aus der Gruppe bestehend aus:
(c) dem Alkan, das 10 Kohlenstoffatome umfasst, und der Carbonsäure, die 6 Kohlenstoffatome umfasst;
(d) dem Alkan, das 12 Kohlenstoffatome umfasst, und der Carbonsäure, die 7 Kohlenstoffatome umfasst; und
(e) dem Alkan, das 14 Kohlenstoffatome umfasst, und der Carbonsäure, die 8 Kohlenstoffatome umfasst.

7. Verfahren nach einem der Ansprüche 1 bis 3, wobei eine Kombination von Carbonsäuren hergestellt wird, umfassend eine erste Carbonsäure und eine zweite Carbonsäure, und das Alkan und die Kombination von Carbonsäuren ausgewählt sind aus der Gruppe bestehend aus:
(f) dem Alkan, das 8 Kohlenstoffatome umfasst, und der ersten Carbonsäure, die 6 Kohlenstoffatome umfasst, und der zweiten Carbonsäure, die 4 Kohlenstoffatome umfasst;
(g) dem Alkan, das 10 Kohlenstoffatome umfasst, und der ersten Carbonsäure, die 5 Kohlenstoffatome umfasst, und der zweiten Carbonsäure, die 7 Kohlenstoffatome umfasst;
(h) dem Alkan, das 12 Kohlenstoffatome umfasst, und der ersten Carbonsäure, die 8 Kohlenstoffatome umfasst, und der zweiten Carbonsäure, die 6 Kohlenstoffatome umfasst;
(i) dem Alkan, das 12 Kohlenstoffatome umfasst, und der ersten Carbonsäure, die 9 Kohlenstoffatome umfasst, und der zweiten Carbonsäure, die 5 Kohlenstoffatome umfasst;
(j) dem Alkan, das 14 Kohlenstoffatome umfasst, und der ersten Carbonsäure, die 6 Kohlenstoffatome umfasst, und der zweiten Carbonsäure, die 8 Kohlenstoffatome umfasst;
(k) dem Alkan, das 9 Kohlenstoffatome umfasst, und der ersten Carbonsäure, die 5 Kohlenstoffatome umfasst, und der zweiten Carbonsäure, die 6 Kohlenstoffatome umfasst;
(1) dem Alkan, das 11 Kohlenstoffatome umfasst, und der ersten Carbonsäure, die 6 Kohlenstoffatome umfasst, und der zweiten Carbonsäure, die 7 Kohlenstoffatome umfasst; und
(m) dem Alkan, das 13 Kohlenstoffatome umfasst, und der ersten Carbonsäure, die 7 Kohlenstoffatome umfasst, und der zweiten Carbonsäure, die 8 Kohlenstoffatome umfasst.

8. Verfahren nach einem der vorhergehenden Ansprüche, wobei das Verfahren einen weiteren Schritt zur Isolierung des Alkans umfasst.

9. Verfahren nach einem der vorhergehenden Ansprüche, wobei die Kolbe-Elektrolyse in einem Elektrolyse-Medium, das Methanol umfasst, durchgeführt wird.

10. Verfahren zur Herstellung wenigstens eines oxidierten Alkanprodukts, wobei das Verfahren Herstellen eines Alkans nach dem Verfahren gemäß einem der Ansprüche 1 bis 9 und anschließendes In-Kontakt-Bringen des Alkans mit wenigstens einem zweiten Mikroorganismus mit der Fähigkeit, das Alkan zum entsprechenden oxidierten Alkanprodukt zu oxidieren, umfasst, wobei das oxidierte Alkanprodukt ausgewählt ist aus der Gruppe bestehend aus entsprechenden Alkoholen, Carbonsäuren und Dicarbonsäuren und der zweite Mikroorganismus ausgewählt ist aus der Gruppe bestehend aus *E. coli, Candida tropicalis, Yarrowia lipolytica* und *Pseudomonas putida.*

## Revendications

1. Procédé de production d'au moins un alcane, le procédé comprenant,
- la production d'au moins un acide carboxylique à partir d'une source de carbone à l'aide d'au moins un micro-organisme, et
- la réalisation d'une électrolyse de Kolbe sur l'acide carboxylique pour produire l'alcane,
l'alcane comprenant au moins 6 atomes de carbone et l'acide carboxylique comprenant au moins 4 atomes de carbone, la source de carbone étant choisie dans le groupe constitué par l'éthanol en combinaison avec au moins une autre source de carbone choisie dans le groupe constitué par un acétate, un propionate, un butyrate, un isobutyrate, un valérate et un hexanoate et le micro-organisme étant choisi dans le groupe constitué par *Clostridium kluyveri* et C. *carboxidivorans.*

2. Procédé selon la revendication 1, le micro-organisme exprimant une protéine de maturation d'hydrogénase et/ou une protéine complexe de transport d'électrons.

3. Procédé selon l'une ou l'autre des revendications 1 et 2, le micro-organisme étant génétiquement modifié et le micro-organisme génétiquement modifié possédant une expression augmentée par rapport au micro-organisme de type sauvage d'au moins une enzyme choisie dans le groupe constitué par une protéine de maturation d'hydrogénase et/ou une protéine complexe de transport d'électrons.

4. Procédé selon l'une quelconque des revendications précédentes, l'alcane étant ramifié ou non ramifié.

5. Procédé selon l'une quelconque des revendications précédentes, l'alcane et l'acide carboxylique étant choisis dans le groupe constitué par :
(a) l'alcane comprenant 6 atomes de carbone et l'acide carboxylique comprenant 4 atomes de carbone ;
(b) l'alcane comprenant 8 atomes de carbone et l'acide carboxylique comprenant 5 atomes de carbone ;
(c) l'alcane comprenant 10 atomes de carbone et l'acide carboxylique comprenant 6 atomes de carbone ;
(d) l'alcane comprenant 12 atomes de carbone et l'acide carboxylique comprenant 7 atomes de carbone ; et
(e) l'alcane comprenant 14 atomes de carbone et l'acide carboxylique comprenant 8 atomes de carbone.

6. Procédé selon l'une quelconque des revendications précédentes, l'alcane et l'acide carboxylique étant choisis dans le groupe constitué par :
(c) l'alcane comprenant 10 atomes de carbone et l'acide carboxylique comprenant 6 atomes de carbone ;
(d) l'alcane comprenant 12 atomes de carbone et l'acide carboxylique comprenant 7 atomes de carbone ; et
(e) l'alcane comprenant 14 atomes de carbone et l'acide carboxylique comprenant 8 atomes de carbone.

7. Procédé selon l'une quelconque des revendications 1 à 3, une combinaison d'acides carboxyliques étant produite comprenant un premier acide carboxylique et un deuxième acide carboxylique et l'alcane et la combinaison d'acides carboxylique étant choisis dans le groupe constitué par :
(f) l'alcane comprend 8 atomes de carbone et le premier acide carboxylique comprend 6 atomes de carbone et le deuxième acide carboxylique comprend 4 atomes de carbone ;
(g) l'alcane comprend 10 atomes de carbone et le premier acide carboxylique comprend 5 atomes de carbone et le deuxième acide carboxylique comprend 7 atomes de carbone ;
(h) l'alcane comprend 12 atomes de carbone et le premier acide carboxylique comprend 8 atomes de carbone et le deuxième acide carboxylique comprend 6 atomes de carbone ;
(i) l'alcane comprend 12 atomes de carbone et le premier acide carboxylique comprend 9 atomes de carbone et le deuxième acide carboxylique comprend 5 atomes de carbone ;
(j) l'alcane comprend 14 atomes de carbone et le premier acide carboxylique comprend 6 atomes de carbone et le deuxième acide carboxylique comprend 8 atomes de carbone ;
(k) l'alcane comprend 9 atomes de carbone et le premier acide carboxylique comprend 5 atomes de carbone et le deuxième acide carboxylique comprend 6 atomes de carbone ;
(1) l'alcane comprend 11 atomes de carbone et le premier acide carboxylique comprend 6 atomes de carbone et le deuxième acide carboxylique comprend 7 atomes de carbone ; et
(m) l'alcane comprend 13 atomes de carbone et le premier acide carboxylique comprend 7 atomes de carbone et le deuxième acide carboxylique comprend 8 atomes de carbone.

8. Procédé selon l'une quelconque des revendications précédentes, le procédé comprenant une étape supplémentaire d'isolement de l'alcane.

9. Procédé selon l'une quelconque des revendications précédentes, l'électrolyse de Kolbe étant réalisée dans un milieu d'électrolyte comprenant du méthanol.

10. Procédé de production d'au moins un produit de type alcane oxydé, le procédé comprenant la production d'un alcane selon le procédé selon l'une quelconque des revendications 1 à 9 et subséquemment la mise en contact de l'alcane avec au moins un deuxième micro-organisme capable d'oxyder l'alcane en produit de type alcane oxydé respectif, le produit de type alcane oxydé étant choisi dans le groupe constitué par des alcools, des acides carboxyliques et des acides dicarboxyliques respectifs et le deuxième micro-organisme étant choisi dans le groupe constitué par *E. coli, Candida tropicalis, Yarrowia lipolytica* et *Pseudomonas putida.*
